# EUROPEAN PATENT APPLICATION

(11) **EP 4 666 951 A1**
(43) Date of publication of application: **24.12.2025**
(21) Application number: 24759471.6
(22) Date of filing: 23.01.2024
(51) Int. Cl.: A61B 6/03, A61B 6/00

(54) **DETECTOR AND CT IMAGING DEVICE**

(30) Priority: 21.02.2023 CN 202310144916
(71) Applicant: Midea Group (Shanghai) Co., Ltd., Shanghai 201702 (CN); Midea Group Co., Ltd., Foshan, Guangdong 528311 (CN)
(72) Inventor: LI, Aaron, Shanghai 201702 (CN); LIU, Bocong, Shanghai 201702 (CN); TANG, Zhiwei, Shanghai 201702 (CN)
(74) Representative: Ran, Handong
(86) International application number: PCT/CN2024/073624
(87) International publication number: WO 2024/174793

(57) **Abstract**

Provided are a detector and a CT imaging device. The detector comprises a detector body (100), which has a detection surface (200). The detection surface (200) is internally provided with a detection region (210) and an auxiliary region (220), which are connected to each other, wherein the detection region (210) is of an integrated structure, and has a first end (211) and a second end (212), which are opposite to each other, and a third end (213) and a fourth end (214), which are opposite to each other, the first end (211), the second end (212), the third end (213) and the fourth end (214) all being located at the edge of the detection surface (200); and the detection region (210) and the auxiliary region (220) fully cover the detection surface (200).

## Description

### CROSS REFERENCE TO RELATED APPLICATIONS

The present disclosure claims priority to the Chinese patent application No. 202310144916.3 filed on February 21, 2023, which may be herein incorporated by reference in its entirety.

### TECHNICAL FIELD

The present disclosure relates to the field of X-ray detection device technologies, and particularly to a detector and a CT imaging device.

### BACKGROUND

Computed tomography (CT), also known as computed tomography scanning, may utilize precisely collimated X-ray beams, γ rays, ultrasound waves, or the like, to perform successive cross-sectional scans around a specific part of a human body together with a detector of extremely high sensitivity. CT may be used for examination of various diseases, and may feature fast scanning speed, clear images, or the like. A CT scanner may include an emission end and a detector. The emission end may be configured to emit radiation. The detector may be configured to receive the radiation.

In the related art, the detector of the CT scanner may need to be configured with not only detection crystals, but also reference modules. The reference modules may occupy installation area of a receiver, which may in turn result in an excessively large size of the detector.

### SUMMARY OF THE DISCLOSURE

The present disclosure aims to solve, at least to a certain extent, a technical problem of excessively large sizes of existing detectors. To this end, the present disclosure provides a detector and a CT imaging device.

According to a first aspect, the detector may be provided in some implementations of the present disclosure. The detector may include a detector body. The detector body may have a detection surface. The detection surface may include a detection area and an auxiliary area that are connected to each other. The detection area may include a first end and a second end opposite to each other, and may include a third end and a fourth end opposite to each other. The first end, the second end, the third end, and the fourth end each may be located at an edge of the detection surface. The detection area and the auxiliary area may fully occupy or completely fill the detection surface.

According to a second aspect, a CT imaging device may be further provided in some implementations of the present disclosure. The CT imaging device may include the above-mentioned detector.

### BRIEF DESCRIPTION OF THE DRAWINGS

In order to more clearly illustrate technical schemes in the present disclosure, the drawings required in description of the embodiments may be briefly introduced below. Obviously, the drawings in the following description may only be some embodiments of the present disclosure. For those of ordinary skills in the art, other drawings may be obtained based on these drawings without creative efforts.
Fig. 1 is a schematic structural diagram of a detector according to some embodiments of the present disclosure.
Fig. 2 is a schematic three-dimensional structural diagram of the detector according to some embodiments of the present disclosure.
Fig. 3 is a schematic diagram of an arrangement approach of an auxiliary area in the detector according to some embodiments of the present disclosure.
Fig. 4 is a schematic diagram of another arrangement approach of the auxiliary area in the detector according to some embodiments of the present disclosure.

Explanation of reference numbers: 100, detector body; 200, detection surface; 210, detection area; 211, first end; 212, second end; 213, third end; 214, fourth end; 220, auxiliary area.

### DETAILED DESCRIPTION

Technical schemes in embodiments of the present disclosure will be described clearly and thoroughly in conjunction with accompanying drawing of embodiments of the present disclosure. Obviously, the described implementations are only part of the implementations of the present disclosure, but not all of them. All other implementations obtained by a person of ordinary skills in the art based on the implementations of the present disclosure without creative efforts may fall in a protection scope of the present disclosure.

It should be noted that, all directional indicators in the embodiments of the present disclosure may only be used to explain a motion state, a relative positional relationship between various components in a specific posture, or the like. If the specific posture changes, the directional indications may change accordingly.

In the present disclosure, unless otherwise definitely specified and limited, the terms "connected", "fixed", or the like should be understood in a broad sense. For example, "fixed" may refer to fixed connections or detachable connections, or integration. In some embodiments, "fixed" may refer to mechanical connections or electrical connections. In some embodiments, "fixed" may refer to direct connections or indirect connections through intermediate mediums, and may refer to internal connection between two components or interaction relationship of the two components, unless otherwise specifically defined. For those of ordinary skills in the art, specific meanings of the above-mentioned terms in the present disclosure may be understood according to specific circumstances.

In addition, the descriptions relating to "first", "second", or the like in the present disclosure are only for purpose of description, and should not be construed as indicating or implying relative importance or implicitly indicating the number of technical features referred to. Therefore, the features preceded by "first", "second" may explicitly or implicitly include at least one of the features. Additionally, the technical schemes of the various embodiments of the present disclosure may be combined with each other, but such combinations must be based on what may be implemented by a person skilled in the art. If a combination of technical schemes leads to contradictions or is unimplementable, such a combination should be considered as not existing, and is not within the protection scope claimed in the present disclosure.

The technical schemes of the present disclosure may be described below with reference to the accompanying drawings and specific embodiments.

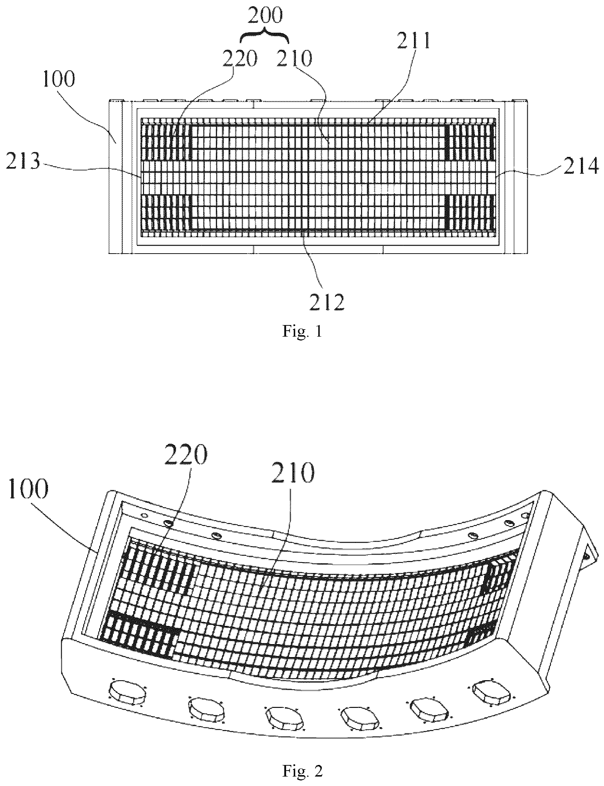

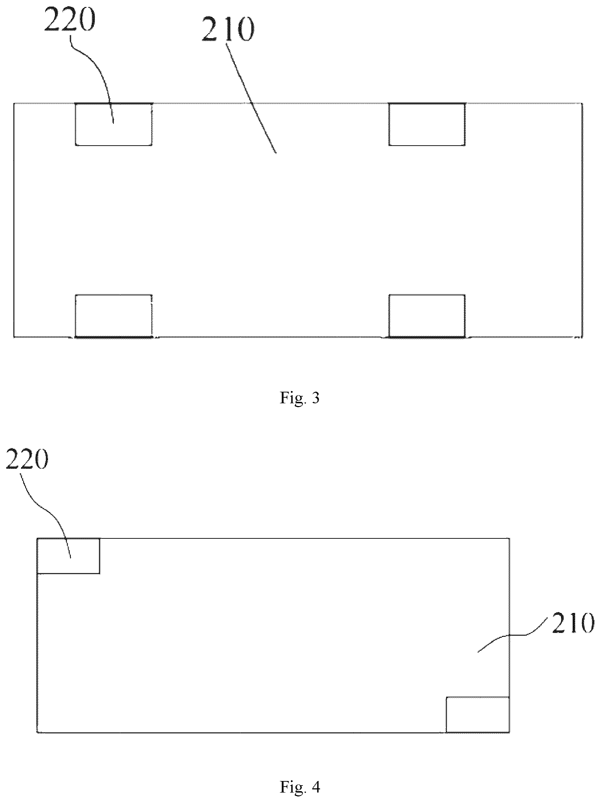

Fig. 1 is a schematic structural diagram of a detector according to some embodiments of the present disclosure. Fig. 2 is a schematic three-dimensional structural diagram of the detector according to some embodiments of the present disclosure. Fig. 3 is a schematic diagram of an arrangement approach of an auxiliary area in the detector according to some embodiments of the present disclosure. Fig. 4 is a schematic diagram of another arrangement approach of the auxiliary area in the detector according to some embodiments of the present disclosure.

As illustrated in Fig. 1 to Fig. 4, the detector according to some embodiments of the present disclosure may include a detector body 100. The detector body 100 may include a detection surface 200. A detection area 210 and an auxiliary area 220 connected to each other may be provided within the detection surface 200. The detection area 210 may include a first end 211 and a second end 212 that are opposite to each other, as well as a third end 213 and a fourth end 214 that are opposite to each other. The first end 211, the second end 212, the third end 213, and the fourth end 214 may all be located at an edge of the detection surface 200. The detection area 210 and the auxiliary area 220 may fully occupy the detection surface 200. This detector may be used in a CT imaging device.

It should be understood that, in addition to the detector, the CT imaging device may also include components such as a tube, a collimator, a control and calculation system, or the like. The tube and the detector may be arranged opposite to each other. The collimator may be located between the tube and the detector. When the CT imaging device is running, the tube may emit X-rays. A subject to be examined may be located between the tube and the detector. After passing through the collimator, the X-rays may pass through a human body and reach the detector. The detector may convert the received X-rays into cross-sectional scanning information of an internal structure of the human body. It should also be understood that, the X-rays emitted by the tube may be a divergent beam that diverges with a light focus as a center. In some embodiments, the X-rays may have a fan-like structure. Therefore, to enable the detector to better receive the X-rays emitted by the tube, the detector may be arranged into an arc-shaped structure. This arrangement may allow various parts of the X-rays emitted by the tube to reach the detector surface relatively perpendicularly, such that an imaging quality of the X-rays on the detector surface may be better. In addition, the X-rays emitted by the tube may pass through the collimator, and form a projection on the detector. The projection may be optimal as a rectangle. The shape of rectangle may enable the CT imaging device to achieve better imaging effects, and may reduce additional radiation dose that a patient has to bear.

It should also be understood that, a defined X-direction, Y-direction, and Z-direction may exist in the CT imaging device. In some embodiments, the Y-direction may be a height direction of the CT imaging device from the tube to the detector. The X-direction may be a direction of a maximum range of X-rays received by the detector. The Z-direction may be a slice direction of the detector. The projection of the detector onto a preset plane in the Y-direction may be a rectangular structure.

The detector body 100 may include the detection surface 200. The detection surface 200 of the detector body 100 may be located on a side of the detector body 100 opposite to the tube. The detection surface 200 may include the detection area 210 and the auxiliary area 220. In some embodiments, the detection area 210 may be provided with detection crystals. There may be a plurality of detection crystals. The plurality of detection crystals may be distributed in the detection area 210 of the detector body 100 along the X-direction and Z-direction of the CT imaging device. In this way, the plurality of detection crystals may be enabled to receive the X-rays emitted by the tube in different directions, such that the X-ray information received by the detector may be complete.

The detection area 210 of the detector body 100 may include the first end 211, the second end 212, the third end 213, and the fourth end 214. In some embodiments, the first end 211 and the second end 212 may be opposite ends of the detection area 210, and the third end 213 and the fourth end 214 may also be opposite ends of the detector. A direction from the first end 211 to the second end 212 may intersect with a direction from the third end 213 to the fourth end 214. Therefore, it may be understood that, the first end 211, the second end 212, the third end 213, and the fourth end 214 of the detection area 210 may respectively match with four sides of the detection surface 200. In some embodiments, the first end 211, the second end 212, the third end 213, and the fourth end 214 of the detection area 210 each may be located at an edge of the detection surface 200. In this way, the first end 211, the second end 212, the third end 213, and the fourth end 214 of the detection area 210 may be enabled to be respectively located at the four sides of the detection surface 200, such that the detection area 210 may have a relatively large extension range.

The detection area 210 may be an integrated structure. In some embodiments, along the direction from the first end 211 to the second end 212, at least part of the detection area 210 may be a continuous structure. This arrangement may enable the detection area 210 to extend entirely between two opposite edges of the detection surface 200. Along the direction from the third end 213 to the fourth end 214, at least part of the detection area 210 may also be a continuous structure. This arrangement may enable the detection area 210 to be extended entirely between other two opposite edges of the detection surface 200. Therefore, it may be ensured that, when a dimension of the detection surface 200 is fixed, the distance from the first end 211 to the second end 212 of the detection area 210 may reach a maximum size, and the distance from the third end 213 to the fourth end 214 may reach a maximum size.

In some embodiments, the direction from the first end 211 to the second end 212 of the detection area 210 may be set as the X-direction of the CT imaging device. Therefore, the first end 211 and the second end 212 of the detection area 210 reaching the edges of the detection surface 200 may enable the detection area 210 to have a maximum X-direction range in the detection surface 200. This may further enable that, when the size of the detector is fixed, the detector of the present disclosure may have a relatively maximum field of view (FOV) for the received X-rays emitted by the tube. Thus, the CT imaging device applying the detector of the present disclosure may be enabled to achieve a larger detection range in a single scan, which may meet a requirement of the CT imaging device for entire scanning of the subject. The direction from the third end 213 to the fourth end 214 of the detection area 210 may be set as the Z-direction of the CT imaging device. Therefore, the third end 213 and the fourth end 214 of the detection area 210 reaching the edges of the detection surface 200 may enable the detection area 210 to have a maximum Z-direction range in the detection surface 200. That is, the number of slices of the detector may be greater, thereby enabling the CT imaging device applying the detector of the present disclosure to be more efficient in detection, which may meet a requirement of the CT imaging device for adequate scanning of major organs of the subject.

The auxiliary area 220 and the detection area 210 of the detection surface 200 may fully occupy the detection surface 200. In this way, at least part of the area of the detection surface 200 may need provision of the auxiliary area 220. Some other components of the detector may be arranged in the auxiliary area 220 of the detection surface 200, or the auxiliary area 200 may be left vacant. When the auxiliary area 220 is vacant, the number of detection crystals may be reduced while the CT imaging device applying the detector of the present disclosure may be enabled to have a relatively maximum scanning range. Therefore, the cost of the CT imaging device may be relatively low. When some other components are arranged in the auxiliary area 220, the detector of the present disclosure may have more abundant functions, and at the same time, a structural arrangement of the CT imaging device may be more diverse.

In the detector proposed in the embodiments of the present disclosure, each of the first end 211, the second end 212, the third end 213, and the fourth end 214 of the detection area 210 may extend to the edges of the detection surface 200. This arrangement may enable the detection area 210 to reach maximum sizes in two directions of the detection surface 200. Therefore, the detector of the present disclosure may be enabled to have a relatively maximum number of slices and a relatively maximum range for receiving the X-rays, such that the CT imaging device applying the detector of the present disclosure may have more diverse detection approaches. Some other components may be arranged in the auxiliary area 220 on the detection surface 200, such that the detector of the present disclosure may have more abundant functions. At the same time, the area of the detection area 210 may also be reduced, thereby reducing the number of the detection crystals, so as to achieve a purpose of reducing the cost of the detector.

In some embodiments, at least part of the first end 211 of the detection area 210 may be opposite to at least part of the second end 212 of the detection area 210. This arrangement may enable a preset straight line extending from the first end 211 to the second end 212 of the detection area 210 (i.e., along the X-direction) to extend on the detection surface 200 at all times while a coordinate of the preset straight line in the Z-direction remains unchanged. In some embodiments, a plurality of detection crystals extending along the X-direction may be arranged on the detection surface 200. The plurality of detection crystals may be arranged to extend continuously along the X-direction. Therefore, the detector may enable, without moving along the Z-direction, the CT imaging device of the present disclosure to have a maximum detection range.

In some embodiments, at least part of the third end 213 of the detection area 210 may be opposite to at least part of the fourth end 214 of the detection area 210. This arrangement may enable a preset straight line extending from the third end 213 to the fourth end 214 of the detection area 210 (i.e., along the Z-direction) to extend on the detection surface 200 at all times while a coordinate of this preset straight line in the X-direction remains unchanged. In some embodiments, the plurality of detection crystals extending along the Z-direction may be arranged on the detection surface 200. The plurality of detection crystals may be arranged to extend continuously along the Z-direction. Therefore, the detector may enable, without moving along the X-direction, the CT imaging device of the present disclosure to have an optimal scanning effect.

In some embodiments, at least part of the edge of the auxiliary area 220 of the present disclosure may be arranged at the edge of the detection surface 200. This may enable the auxiliary area 220 of the detection surface 200 to be located at an edge position of the detection surface 200. Correspondingly, the detection area 210 of the detection surface 200 may occupy a central position of the detection surface 200. It should be understood that, when the CT imaging device is detecting the subject, the subject may lie flat on a movable bed. A middle part of a body of the subject may be opposite to or match with a middle part of the detector. Therefore, the detection area 210 of the present disclosure may be located in the middle part of the detection surface 200. This arrangement may allow for detection of important organs of the subject, such as the heart, the lungs, and the stomach, when the subject is lying normally on the movable bed, thereby making a detection process of the CT imaging device using the detector of the present disclosure more convenient.

Of course, in other embodiments, the auxiliary area 220 of the detection surface 200 may also be arranged at a position relatively close to the middle of the detection surface 200, such that the side edges of the auxiliary area 220 may not contact the edges of the detection surface 200. This arrangement may enable the auxiliary area 220 of the detection surface 200 to be located within the detection area 210.

In some embodiments, the auxiliary area 220 of the present disclosure may be arranged at a corner position of the detection surface 200. In some embodiments, two side edges of the auxiliary area 220 of the detection surface 200 may be arranged at the edges of the detection surface 200. This arrangement may make the auxiliary area 220 to be relatively more deviated from the middle part of the detection surface 200, and the middle part of the detector may all be used for arranging the detection area 210, such that the detection area 210 may be relatively concentrated on the detection surface 200.

Of course, in some other embodiments, when only one side edge of the detection surface 200 is located at the edge position of the detection surface 200, the auxiliary area 220 may be arranged at a position relatively close to a corner of the detection surface 200, such that the auxiliary area 220 may be relatively far from the middle area of the detection surface 200. In some embodiments, when a side edge of the auxiliary area 220 is in contact with an edge of the detection surface 200 in the Z-direction, the auxiliary area 220 may be arranged close to an edge of the detection surface 200 in the X-direction; when a side edge of the auxiliary area 220 is in contact with an edge of the detection surface 200 in the X-direction, the auxiliary area 220 may be arranged close to an edge of the detection surface 200 in the Z-direction. Of course, in some other embodiments, if the components to be arranged in the auxiliary area 220 need to be placed in the middle part of the detection surface 200, the auxiliary area 220 may also be arranged at a center line of the detection surface 200. It should be understood that, the position of the auxiliary area 220 may not be fixed, and may be flexibly adjusted according to installation positions required by different components arranged in the auxiliary area 220.

In some embodiments, a reference module may be specifically arranged in the auxiliary area 220 of the detection surface 200 of the present disclosure. The reference module may specifically also be a detection crystal. Since the auxiliary area 220 of the detection surface 200 is located at a corner position of the detection surface 200, when the CT imaging device scans the subject, the auxiliary area 220 of the detection surface 200 may be offset from the subject. In this way, the X-rays emitted by the tube and matching with the auxiliary area 220 may not pass through the subject, such that the reference module in the detection area 210 may receive the original X-rays emitted by the tube, and thus parameters of the X-rays from the tube may be obtained. However, the X-rays emitted by the tube and matching with the detection area 210 may pass through the subject and then be received by the detection crystals in the detection area 210. Therefore, the X-rays received by the detection crystals in the detection area 210 may be the X-rays absorbed by the body of the subject. By comparing the X-rays received by the detection crystals in the detection area 210 with those received by the reference module in the auxiliary area 220, the amount of the X-rays absorbed by the body of the subject may be accurately obtained, thereby facilitating acquisition of a physical condition of the subject.

Of course, in some other embodiments, to make the functions of the detector of the present disclosure more abundant, components in the auxiliary area 220 of the detection surface 200 may be arranged in the auxiliary area 220 in a detachable manner. In this way, when the detector of the present disclosure requires to be configured with different functions, such a requirement may be accomplished by replacing the components in the auxiliary area 220. In this way, the functions that the detector of the present disclosure may implement are enriched, and the functions of the CT imaging device applying the detector of the present disclosure may be further enriched.

In some embodiments, it should be understood that, the structure of the detection crystal in the detector may be a rectangular-like structure. To enable the detection effect of the detector of the present disclosure to be better, the detection surface 200 of the detector body may be arranged as a rectangular-like arc surface. In some embodiments, a projection of the detection surface 200 formed on a preset plane opposite to the detection surface 200 may be a rectangular shape. This may enable the detection crystals in the detection area 210 to be arranged in an orderly manner along the X-direction and the Z-direction of the CT imaging device, such that more detection crystals with standard structures may be arranged in the detection area 210, and space waste on the detection surface 200 of the detector body may be avoided.

In some embodiments, it should also be understood that, if the structure of the detection crystal is a cylindrical structure, to make the detection effect of the detector of the present disclosure better, the detection surface 200 of the detector body may be configured as an elliptical-like arc surface. In some embodiments, a projection of the detection surface 200 formed on the preset plane opposite to the detection surface 200 may be an elliptical shape. This may also enable the detection crystals in the detection area 210 to be arranged in an orderly manner along the X-direction and Z-direction of the CT imaging device, and more detection crystals with cylindrical structures may be arranged in the detection area 210, and space waste on the detection surface 200 of the detector body may be avoided. Therefore, it should be understood that, the shape of the detection surface 200 of the detector may be flexibly adjusted according to a structure of the detection crystal, such that the shape of the detection surface 200 may match that of the detection crystal.

In some embodiments, it should be understood that, the reference module in the auxiliary area 220 may essentially be a detection crystal as well. Therefore, to enable relatively more detection modules to be arranged in the auxiliary area 220, the edges of the projections formed by the detection area 210 and the auxiliary area 220 of the detection surface 200 on the preset plane may all be straight edges. This may allow relatively more detection crystals in the detection area 210 and relatively more reference modules in the auxiliary area 220 to be arranged.

In some embodiments, to make the parameters of the X-rays obtained by the reference module arranged in the auxiliary area 220 of the present disclosure more accurate, the number of auxiliary areas 220 may be set to more than one. The more than one auxiliary areas 220 may be arranged at intervals in various parts of the detection surface 200 of the detector body. Therefore, the reference modules in the more than one auxiliary areas 220 may obtain the parameters of the X-rays emitted by the tube from different directions. An average value may be obtained by integrating the X-ray parameters obtained by the reference modules on various auxiliary areas 220, thereby reducing an error caused by different parameters of the X-rays from different directions.

It should be understood that, among the X-rays emitted by the tube, parameter characteristics of relatively adjacent X-rays may be relatively close, while differences in parameters among relatively distant X-rays may be relatively great. Therefore, the more than one auxiliary areas 220 of the detection surface 200 may also be distributed relatively dispersedly on the detection surface 200, such that small areas centered on the more than one auxiliary areas 220 may be formed on the detection surface 200. The X-rays matching with any same small area may be relatively adjacent, and the parameter characteristics of this part of the X-rays may be relatively similar. The X-ray parameters obtained by the detection crystals in the detection area 210 within any small area may be compared with those obtained by the reference modules in the auxiliary area 220 within the same small area, thereby making scanning results of the CT imaging device applying the detector of the present disclosure more accurate.

In some embodiments, the number of the auxiliary areas 220 of the detection surface 200 may be set to four. The four auxiliary areas 220 may be respectively arranged at four corners of the detection surface 200, and structures of the four auxiliary areas 220 may be set to be consistent, such that the four auxiliary areas 220 may be arranged symmetrically relative to the center line of the detection surface 200. By arranging the four auxiliary areas 220 at the four corners of the detection surface 200, the four auxiliary areas 220 may be enabled to receive relatively farthest apart portions of the X-rays emitted by the tube, thereby enhancing a reference effect of the data collected by the reference module.

In some embodiments, a distance from the first end 211 to the second end 212 of the detection area 210 of the detection surface 200 in the present disclosure may be set to be greater than 600mm. In this way, the size of the detector of the present disclosure in the X-direction may be enabled to meet most detection requirements. At the same time, a distance from the third end 213 to the fourth end 214 of the detection area 210 of the detection surface 200 may be set to be greater than 160mm. In this way, the size of the detector of the present disclosure in the Z-direction may be enabled to meet most detection requirements, thereby expanding an application range of the CT imaging device applying the detector of the present disclosure.

A CT imaging device may also be proposed according to some embodiments of the present disclosure. The CT imaging device may include the above-mentioned detector.

In some implementations of the present disclosure, the first end, the second end, the third end, and the fourth end of the detection area may extend to the edge of the detection surface. This may enable the detection area to reach maximum sizes in two directions within the detection surface, such that the detector according to some embodiments of the present disclosure may have a relatively maximum number of slices and a relatively maximum range for receiving X-rays. Thus, the CT imaging device applying the detector of the present disclosure may have more diverse detection approaches. Some other components may be arranged in the auxiliary area on the detection surface, such that the detector of the present disclosure may have more abundant functions. At the same time, the area of the detection area may also be reduced, thereby reducing the number of the detection crystals, so as to achieve a purpose of reducing the cost of the detector.

In the description of the present specification, descriptions referring to terms "one embodiment", "some embodiments", "example", "specific example", "some examples", or the like may mean that, a specific feature, structure, material, or characteristic described in conjunction with the implementations or examples may be included in at least one embodiment or example of the present disclosure. In the present specification, schematic representations of the above-mentioned terms do not necessarily refer to a same embodiment or example. Moreover, specific features, structures, materials, or characteristics described may be combined in any suitable manner in any one or more embodiments or examples. In addition, those skilled in the art may combine and integrate different embodiments or examples described in the present specification.

## Claims

1. A detector, comprising:
a detector body (100), comprising a detection surface (200); wherein
the detection surface (200) comprises a detection area (210) and an auxiliary area (220) that are connected to each other;
the detection area (210) comprises a first end (211) and a second end (212) that are opposite to each other, as well as a third end (213) and a fourth end (214) that are opposite to each other;
each of the first end (211), the second end (212), the third end (213), and the fourth end (214) is located at an edge of the detection surface (200); and
the detection area (210) and the auxiliary area (220) fully occupy the detection surface (200).

2. The detector as claimed in claim 1, wherein
at least part of the first end (211) is opposite to at least part of the second end (212), and at least part of the third end (213) is opposite to at least part of the fourth end (214).

3. The detector as claimed in claim 2, wherein
at least part of an edge of the auxiliary area (220) is located at an edge of the detection surface (200).

4. The detector as claimed in claim 3, wherein
the auxiliary area (220) is arranged at a corner of the detection surface (200).

5. The detector as claimed in any one of claims 1-4, wherein
the detector comprises a preset plane opposite to the detection surface (200), and a projection of the detection surface (200) formed on the preset plane is a rectangle.

6. The detector as claimed in claim 5, wherein
a projection of the auxiliary area (220) formed on the preset plane is also a rectangle, and two adjacent edges of the auxiliary area (220) are located at edges of the detection surface (200).

7. The detector as claimed in claim 6, wherein
the number of the auxiliary areas (220) is more than one, and the more than one auxiliary areas (220) are arranged at intervals on the detection surface (200).

8. The detector as claimed in claim 7, wherein
the number of the auxiliary areas (220) is four, and the four auxiliary areas (220) are arranged at four corners of the detection surface (200) respectively.

9. The detector as claimed in claim 8, wherein
the four auxiliary areas (220) are arranged symmetrically along a center line of the detection surface (200).

10. The detector as claimed in any of claims 6-9, further comprising a reference module, wherein
the reference module is arranged in the auxiliary area (220).

11. The detector as claimed in claim 1, wherein
within the detection surface (200), a distance from the first end (211) to the second end (212) is greater than 600mm, and a distance from the third end (213) to the fourth end (214) is greater than 160mm.

12. A CT imaging device, comprising the detector as claimed in any one of claims 1-11.
